# DEMANDE DE BREVET EUROPEEN

(11) **EP 0 526 349 A1**
(43) Date de publication de la demande: **03.02.1993**
(21) Numéro de dépôt: 92402216.3
(22) Date de dépôt: 03.08.1992
(51) Int. Cl.: B65F 1/14, A61B 19/02, B30B 9/30

(54) **Container d'objets souillés**

(30) Priorité: 02.08.1991 FR 9109893
(71) Demandeur: Charles, Daniel, F-21160 Couchey (FR)
(72) Inventeur: Charles, Daniel, F-21160 Couchey (FR)
(74) Mandataire: Bruder, Michel

(57) **Abrégé**

La présente invention concerne un container d'objets souillés particulièrement destiné aux établissements hospitaliers, comportant un bac (1) muni d'un couvercle (8) et pourvu extérieurement de moyens de manutention, des moyens de blocage étant agencés à l'intérieur dudit bac (1) pour coopérer avec au moins une plaque de double fond (11) destinée à venir comprimer par le dessus la masse d'objets souillés se trouvant dans ledit bac (1) de sorte à dégager, au-dessus de ladite plaque de double fond (11), un volume supplémentaire pour la récupération d'autres objets souillés, ledit container étant caractérisé en ce que lesdits moyens de blocage sont constitués par un nombre variable de linguets élastiques (13), agencés sur le pourtour d'un plateau (12) faisant office de plaque de fond (11), lesdits linguets (13) s'évasant de bas en haut vers l'extérieur dudit plateau (12) pour venir s'engager sous au moins une rive (10) périphérique, continue ou discontinue, s'étendant au moins partiellement sur les parois internes dudit bac (1).

## Description

La présente invention concerne un container d'objets souillés particulièrement destiné aux établissements hospitaliers.

L'élimination des déchets hospitaliers tels que compresses, seringues et autres objets souillés s'effectue généralement par l'intermédiaire de containers en matières plastiques imperforables qui sont munis d'un couvercle étanche et inviolable qu'on ne peut enlever sans l'aide d'un outil adapté. Ces containers, d'une capacité d'environ 30 litres, 60 litres ou plus, sont, une fois remplis, disposés sur une plate-forme, palettisés, puis transportés vers un incinérateur où l'ensemble des containers est mis à brûler.

Or, du fait que les containers peuvent être remplis d'objets souillés présentant une grande variété, leur poids varie assez largement ; on comprend notamment qu'un container rempli de compresses soit beaucoup plus léger qu'un container rempli d'objets beaucoup plus denses et compacts (champs opératoires, lames, etc...). Par ailleurs, il est important qu'un chirurgien, un infirmier ou une femme de service ne puisse pas se blesser en introduisant dans le container l'objet souillé dont il désire se débarrasser, en particulier en raison des risques de piqûres par des aiguilles et autres objets coupants (lames, scapels, etc..) pouvant déjà se trouver à l'intérieur du container ; c'est pourquoi il est fortement déconseillé de tasser le contenu d'un container, ce qui contribuerait néanmoins à diminuer la place perdue dans ce dernier. Il s'est ainsi avéré utile de développer des containers de capacité double, voire triple, de ce la capacité actuelle des containers. Une telle solution n'est cependant pas toujours acceptable car l'encombrement d'un container de plus de 100 litres pose des problèmes de manutention importants.

On connaît également, par le biais des brevets US-A-4 462 310 et US-A-4 760 784, des containers pour le stockage de produits contamines, notamment radioactifs, qui sont associes à des moyens de compactage permettant de dégager un nouvel espace de remplissage à la partie supérieure desdits containers. A cet effet, le brevet US-A-4 462 310 propose un plateau de compactage destine à être inséré dans un container cylindrique, métallique et rigide ; ce container comporte intérieurement un ensemble de tiges filetées verticales qui coopèrent avec des pattes de blocage métalliques fixées sur le dessus dudit plateau de compactage. De même, le brevet US-A-4 760 784 concerne un dispositif de compactage analogue comportant des moyens de blocage du plateau différents de ceux préconisés dans le brevet précédemment cité ; à cet égard, le plateau de compactage comporte des taquets de blocage élastiques, par exemple en un matériau élastomère, dont la compression contre la paroi interne d'un container cylindrique rigide de même section droite suffit au maintien dudit plateau à une hauteur quelconque à l'intérieur dudit container.

Ces deux solutions antérieures au compactage d'objets peu denses dans des containers de stockage de déchets présentent plusieurs inconvénients importants, et notamment :
- étant fabriques pour contenir des déchets principalement radioactifs, ces containers sont destinés au stockage et non à l'élimination des déchets ; or, le seul fait que ces containers soient métalliques, ou nécessitent des moyens de blocage ou de compaction qui, par principe, soient le plus endurants possible, interdit leur utilisation en tant que containers d'élimination de déchets simplement souillés. Cette impossibilité est même aggravante dans le cas de déchets hospitaliers qui devraient forcément être extraits desdits containers, par nature indestructibles, avant d'être brûles ; or, il est impératif que l'élimination de déchets hospitaliers pouvant conduire à une contamination quelconque soit assurée dans des conditions de sécurité et d'hygiène les plus draconiennes.
- d'un autre côte, les moyens de blocage employés pour la fixation des plateaux de compactage à l'intérieur des containers sont à l'évidence complexes et coûteux à fabriquer. En outre, les moyens de blocage par taquets élastiques élastomères nécessitent que le container soit rigide ; l'autre possibilité connue, associant tiges filetées et plaques, est quant à elle très lourde à mettre en oeuvre.

La présente invention vise à remédier à ces inconvénients en proposant un container d'objets souilles particulièrement destiné aux établissements hospitaliers, comportant un bac en matière plastique imperforable muni d'un couvercle et pourvu extérieurement de moyens de manutention, des moyens de blocage étant agences à l'intérieur dudit bac pour coopérer avec au moins une plaque de double fond destinée à venir comprimer par le dessus la masse d'objets souilles se trouvant dans ledit bac de sorte à dégager, au-dessus de ladite plaque de double fond, un volume supplémentaire pour la récupération d'autres objets souilles, ce container étant caractérisé en ce que lesdits moyens de blocage sont constitues par un nombre variable de linguets élastiques, agencés sur le pourtour de ladite plaque de double fond pour venir s'engager sous au moins une rive périphérique, continue ou discontinue, s'étendant au moins partiellement sur les parois internes dudit bac.

Préférentiellement, les linguets s'évasent de bas en haut vers l'extérieur de la plaque de double fond, ci-après également dénommée plateau.

Par ce moyen, on utilise au mieux la capacité d'un container sans, toutefois, faire prendre aucun risque à l'utilisateur qui, du fait qu'il se contente d'appuyer sur le dessus de la plaque de double fond, n'est jamais en contact avec les objets souillés se trouvant au fond dudit bac.

Bien entendu, la matière choisie pour la réalisation de ladite plaque est identique à celle normalement utilisée pour le bac, c'est-à-dire consiste en une matière plastique imperforable.

Eventuellement, le bac peut être muni d'une série de rives superposées les unes au dessus des autres sur les parois internes du bac, à la manière d'une crémaillère, de sorte que leur hauteur varie par rapport au fond dudit bac ; cette disposition de l'invention permet d'adapter le tassement des objets souilles comprimés par le double fond en fonction de leur densité. Par exemple, pour des objets peu denses telles que des compresses, la plaque de double fond pourra être bloquée sous la rive périphérique la plus basse prévue sur les parois intérieures du bac.

D'autres caractéristiques et avantages de la présente invention ressortiront mieux de la description qui va suivre d'un mode d'exécution d'un container d'objets souillés donne à titre d'exemple non limitatif en référence aux dessins annexés sur lesquels :
- la figure 1 est une vue en perspective éclatée dudit container montrant notamment le bac, son couvercle et une plaque de double fond pouvant coopérer avec des rives de blocage aménagées à cet effet le long du bac,
- la figure 2 est une vue en coupe sagittale du container représenté sur la figure 1, où on a représente une plaque de double fond mise en place pour comprimer des objets souillés à la partie inférieure de son bac,
- la figure 3 est un exemple de palettisation horizontale bidirectionnelle de containers identiques à celui représenté sur les figures 1 et 2.

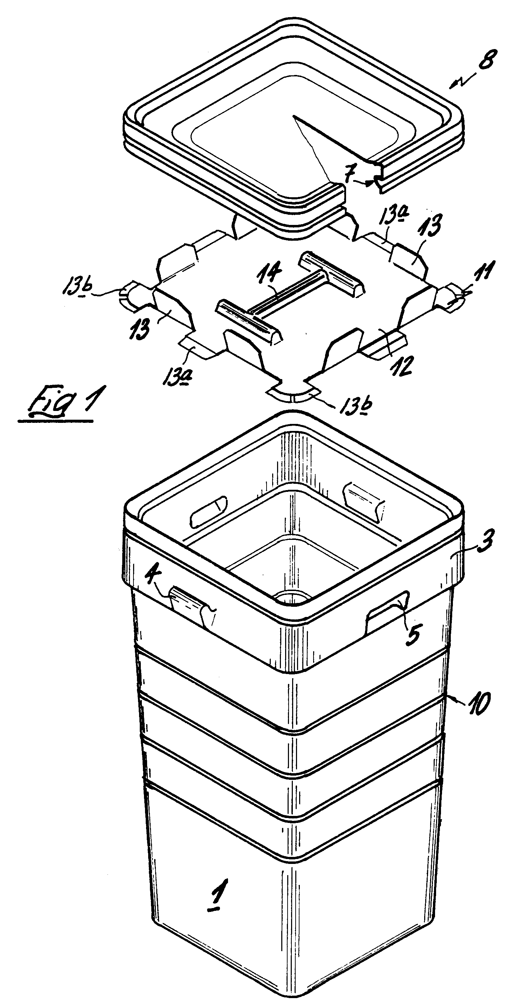

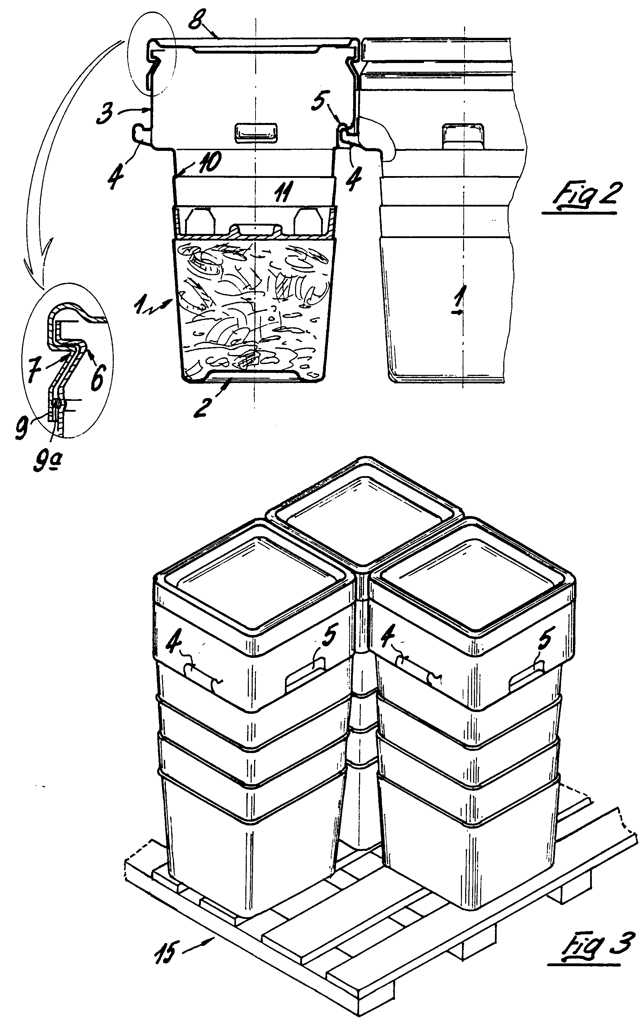

Le container représenté sur les figures 1 et 2 est constitue par un bac 1 de forme tronconique s'évasant de bas en haut depuis un fond 2 de section rectangulaire légèrement bombé vers le haut. Ce bac 1 comporte, à environ les trois-quarts de sa hauteur, un renflement 3 faisant saillie vers l'extérieur destine à faciliter la préhension dudit bac 1 par le personnel de manutention. Sur ce renflement 3 sont aménagées, sensiblement au milieu de deux flans adjacents du bac 1, des griffes 4 de palettisation et de centrage destinées à venir s'engager dans des encastrements 5 leur correspondant sur les deux autres flans dudit bac 1. Au-dessus du renflement 3, et juste à la limite supérieure du bac 1, une gorge périphérique 6 sert de logement à un clip 7 aménagée sur la circonférence d'un couvercle 8 destiné à venir coiffer le bac 1. Ce couvercle 8 est bombé vers l'intérieur suivant une forme correspondant à celle du fond 2 de manière à assurer une assise correcte à un container qui serait placé sur le dessus dudit couvercle 8. Afin d'assurer une étanchéité correcte au container, le clip 7 est prolongé vers le bas par une jupe souple 9 venant épouser les flans du bac 1, juste en-dessous de la gorge périphérique 6, un joint torique 10 pouvant, en outre, être le cas échéant intercalé entre le bas de ladite jupe souple 9 et les flans dudit bac 1.

Suivant la présente invention, le bac 1 est également pourvu de moyens de blocage, du type d'une série de rives 10 superposées les unes au dessus des autres sur les parois internes du bac 1 à la manière d'une crémaillère, pour coopérer avec une plaque de double fond 11 destinée à venir comprimer par le dessus la masse d'objets souillés se trouvant dans ledit bac 1. Dans la variante représentée sur les figures, cette plaque de double fond 11 est constituée par un plateau 12 rectangulaire, homothétique de la section droite du bac 1, sur le pourtour duquel est agence un nombre variable de linguets 13 élastiques, s'évasant de bas en haut vers l'extérieur dudit plateau 12 ; une fois en place, conformément à la figure 2, les linguets viennent s'engager sous l'une des rives 10 prévues à l'intérieur du bac 1. On notera que l'élasticité procurée par les linguets 13 rend la plaque de double fond 11 particulièrement stable et évite son décrochement sous l'effet de la décompression des objets souillés se trouvant en partie inférieure du bac 1. En outre, il est prévu d'aménager des jupes 13a et 13b, respectivement entre deux linguets 13 d'un bord du plateau 12 et aux coins dudit plateau 12 ; ces jupes 13a, 13b ont pour rôle de venir "lécher" la paroi interne du bac 1 de manière à dégager les rives 10 de tout encombrement dû à la remontée des objets souillés dans les interstices qui seraient sinon laisses libres entre les linguets 13 et ladite paroi interne du bac 1. On notera néanmoins que ces jupes 13a, 13b sont tout-à-fait facultatives et que, dans tous les cas de figures, l'encombrement éventuel de l'espace situe entre deux linguets 13 ne nuit pas au blocage du plateau 12 sous la rive 10 concernée ; en effet, le nombre de linguets 13 prévus sur le pourtour dudit plateau 12 est suffisant pour rendre peu probable le cas d'un encombrement complet desdits linguets 13. En outre, même en cas de relâchement du plateau 12 vers le haut, l'élasticité des linguets 13 vers l'extérieur du bac 1 est suffisante pour que lesdits linguets 13 viennent se bloquer sous la rive 10 immédiatement supérieure aménagés le long du bac 1.

Suivant une caractéristique complémentaire de la présente invention, la face supérieure du plateau 12 de la plaque de double fond 11 est pourvu de moyens de préhension 14 adoptant la forme d'un H ; cette forme n'est pas obligatoire et on pourrait tout aussi bien prévoir des moyens de préhension 14 de la plaque de double fond 11 agencés différemment.

Suivant une autre caractéristique de l'invention, la surface de la section délimitée par le plateau 12 de la plaque de double fond 11 est sensiblement égale à la surface de la section droite du bac 1 interceptée par le plan horizontal passant par ledit plateau 12 lorsque la plaque de double fond 11 se trouve à sa position la plus basse à l'intérieur dudit bac 1 ; de la sorte, il existe dans cette position un jeu minimum entre ledit plateau 12 et les parois intérieures du bac 1. De ce fait, la forme tronconique du bac 1 pourrait rendre difficile l'accrochage des linguets 13 sous les rives 10 les plus hautes ; c'est pourquoi, selon l'invention, les linguets élastiques 13 présentent, par rapport au plateau 12 de la plaque de double fond 11, une inclinaison vers l'extérieur d'angle supérieur à l'angle dont sont inclines les flans du bac 1.

L'utilisation du container représenté sur les figures 1 et 2 est la suivante : lorsque l'utilisateur s'aperçoit que le bac 1 est complètement rempli, il en retire le couvercle 8 et, en utilisant une plaque de double fond 11 telle que celle proposée plus haut, il vient presser sur le dessus des objets souilles se trouvant dans le bac 1 de manière à les tasser le plus possible dans la partie inférieure dudit bac 1. Le blocage de la plaque de double fond 11 peut, grâce aux diverses rives 10 de hauteurs différentes, être effectué progressivement. On comprend également que la hauteur de la plaque de double fond 11 à l'intérieur du bac 1 dépend dans une large mesure de la force de l'utilisateur. Par contre, le blocage est dans tous les cas assure d'une manière stable par le biais des linguets élastiques 13 qui contribuent à repousser en permanence vers le bas les objets souilles comprimes (compresses notamment) qui auraient, dans certains cas, une tendance normale à se distendre. A cet égard, on notera que le système à linguets 13, souples et élastiques, choisi pour constituer la plaque de double fond 11 procure un autoblocage de cette dernière le long de la crémaillère formée par la superposition verticale des rives 10, et évite sa remontée vers le haut au fur et à mesure que l'on enfonce le plateau 12 vers le fond du bac 1.

Suivant une autre caractéristique de la présente invention, il est prévu de proposer, avec les containers, un outil spécifique motorisé chargé d'appuyer sur la plaque de double fond 11 au moment où on l'introduit dans l'intérieur du bac 1, de manière à comprimer le plus possible les objets souilles s'y trouvant et à regagner un large espace disponible au dessus du plateau 12 de la plaque de double fond 11. Le volume ainsi gagne par tassement peut être de nouveau exploite pour y jeter des déchets, l'opération précédente pouvant, le cas échéant, être recommencée pour les containers dont la capacité est la plus importante.

Une fois chargés, les containers ayant subi une ou plusieurs fois l'opération précédente sont transportés sur une plate-forme 15 (cf. figure 3) et, par le biais des griffes 4 et des encastrements 5 prévus deux à deux sur leurs flans, ils peuvent être emboîtés les uns dans les autres pour former un assemblage horizontal indissociable. En outre, une fois qu'une première rangée est établie sur la plate-forme, il est facile d'y empiler une seconde voire une troisième rangée, la forme spécifique bombée vers l'intérieur du couvercle 8 d'un container facilitant une assise correcte du container disposé à son aplomb. Ainsi palettisée puis gerbée, la plate-forme est globalement emmenée vers l'incinérateur afin d'y être brûlée.

L'invention n'est bien entendu pas limité par la description qui vient d'être donnée ni par les dessins annexés mais s'étend à toute réalisation d'un container pourvu d'un double fond mobile réalisé dans son esprit.

## Revendications

**1 -** Container d'objets souillés particulièrement destiné aux établissements hospitaliers, comportant un bac (1) muni d'un couvercle (8) et pourvu extérieurement de moyens de manutention, des moyens de blocage étant agencés à l'intérieur dudit bac (1) pour coopérer avec au moins une plaque de double fond (11) destinée à venir comprimer par le dessus la masse d'objets souillés se trouvant dans ledit bac (1) de sorte à dégager, au-dessus de ladite plaque de double fond (11), un volume supplémentaire pour la récupération d'autres objets souillés, ledit container étant caractérisé en ce que lesdits moyens de blocage sont constitués par un nombre variable de linguets élastiques (13), agencés sur le pourtour d'un plateau (12) faisant office de plaque de fond (11), lesdits linguets (13) s'évasant de bas en haut vers l'extérieur dudit plateau (12) pour venir s'engager sous au moins une rive (10) périphérique, continue ou discontinue, s'étendant au moins partiellement sur les parois internes dudit bac (1).

**2 -** Container selon la revendication précédente, caractérisé en ce que le bac (1) est muni d'une série de rives (10) superposées les unes au dessus des autres sur les parois internes du bac (1) à la manière d'une crémaillère, de sorte que leur hauteur varie par rapport au fond dudit bac (1).

**3 -** Container selon l'une quelconque des revendications précédentes, caractérisé en ce que la surface de la section délimitée par le plateau (12) de la plaque de double fond (11) est sensiblement égale à la surface de la section droite du bac (1) interceptée par le plan horizontal passant par ledit plateau (12) lorsque la plaque de double fond (11) se trouve à sa position la plus basse à l'intérieur dudit bac (1).

**4 -** Container selon l'une quelconque des revendications précédentes, caractérisé en ce que les linguets élastiques (13) présentent, par rapport au plateau (12) de la plaque de double fond (11), une inclinaison vers l'extérieur d'angle supérieur à l'angle dont sont inclinés les flans du bac (1).

**5 -** Container selon l'une quelconque des revendications précédentes, caractérisé en ce que des jupes (13a) et (13b) sont respectivement aménagées entre deux linguets (13) d'un bord du plateau (12) et aux coins dudit plateau (12), ces jupes (13a, 13b) venant lécher la paroi interne du bac (1) de manière à dégager les rives (10) de tout encombrement éventuel.

**6 -** Container selon l'une quelconque des revendications précédentes, caractérisé en ce que la face supérieure du plateau (12) de la plaque de double fond (11) est pourvue de moyens de préhension (14).

**7 -** Container selon l'une quelconque des revendications précédentes, caractérisé en ce que le bac (1) et la plaque de double fond (11) sont réalisés par moulage d'une matière plastique imperforable.

**8 -** Container selon l'une quelconque des revendications précédentes, caractérisé en ce qu'il présente une section droite carrée ou rectangulaire, et en ce qu'il est muni d'au moins une griffe (4) externe aménagée sur au moins l'une des faces du bac (1) pour venir s'engager dans un encastrement (5) prévu sur au moins une face du bac (1) d'un autre container.

**9 -** Container selon la revendication précédente, caractérisé en ce qu'il comporte des griffes (4) sur deux faces adjacentes et des encastrements (5) leur correspondant sur ses deux autres faces.
